# EUROPÄISCHE PATENTANMELDUNG

(11) **EP 1 329 720 A2**
(43) Veröffentlichungstag der Anmeldung: **23.07.2003**
(21) Anmeldenummer: 02029119.1
(22) Anmeldetag: 24.12.2002
(51) Int. Cl.: G01N 33/68

(54) **Verfahren zum Nachweis von pathogenen Prionenproteinen durch Massenspektroskopie**

(30) Priorität: 17.01.2002 DE 10201777
(71) Anmelder: Aventis Behring GmbH, 35002 Marburg (DE)
(72) Erfinder: Lengsfeld, Thomas, Dr., 35041 Marburg (DE)

(57) **Zusammenfassung**

Es wird ein Verfahren zum Nachweis von pathogenen Prionenproteinen in einer Probe einer Körperflüssigkeit humanen oder tierischen Ursprungs, enthaltend ein PrP-Protein, das eine erste natürliche, nicht-pathologische Konformation, PrP^{c}, und eine zweite pathologische Konformation, PrP^{Sc} genannt, einnimmt, beschrieben bei dem man
- in einer Probe einer Körperflüssigkeit, welche auch chemisch modifiziert sein kann,
- die Prionenproteine mit einem chemischen Agens unter Bildung kovalenter Bindungen umsetzt und
- die dadurch chemisch modifizierten Prionenproteine massenspektroskopisch untersucht, wobei bei Vorliegen von pathogenen Prionen im Massenspektrum mindestens ein weiterer Peak beobachtet wird.

## Beschreibung

Die Erfindung betrifft ein Verfahren zum Nachweis von pathogenen Prionenproteinen in einer Probe einer Körperflüssigkeit humanen oder tierischen Ursprungs durch ein massenspektroskopisches Verfahren.

Prionerkrankungen wie z. B. die Creutzfeldt-Jakob-Erkrankung (CJD) können sowohl durch vererbte genetische Defekte entstehen als auch über noch nicht vollständig verstandene Infektionswege erworben werden. Darüber hinaus treten sie auch als spontane, sogenannte sporadische Formen auf, für die eine somatische Mutation im Gen für das Prionenprotein postuliert wird (Prusiner, Proc. Natl. Acad. Sci. U.S.A., 95, 13363-13383 (1998)). latrogene Infektionswege entstehen zum Beispiel durch Behandlung mit prionen-verseuchten Wachstumshormonen, Geschlechtshormonen oder Hornhaut- und Hirnhauttransplantaten. Auch die Verwendung von nicht ausreichend sterilisiertem chirurgischem Material stellt eine mögliche Infektionsquelle dar.

Die 33 bis 35 kD großen Prionenproteine (abgekürzt PrP) kommen in einer natürlichen physiologischen Isoform (PrP^{c}) sowie in einer pathologisch infektiösen Isoform (PrP^{Sc}) vor, wobei die infektiöse Isoform durch eine Umfaltung der Sekundär- und Tertiärstruktur aus der nichtinfektiösen physiologischen Form entsteht. Das PrP^{Sc} ist höchstwahrscheinlich die einzige stoffliche Komponente der Prionen, die für die Transmission und die Pathogenese der Prionenerkrankungen benötigt wird (Prusiner, Proc. Natl. Acad. Sci. U.S.A., 95, 13363-13383 (1998)).

Aus Prusiner et al., Cell 38, 127 (1984) sowie Biochemistry 21, 6942 (1982) ist es bereits bekannt, dass Prionenproteine einer partiellen Proteolyse zugänglich sind. In der Folgezeit hat es sich gezeigt, dass PrP^{C} nahezu vollständig einer Proteolyse zugänglich ist, wohingegen PrP^{Sc} sich lediglich bis zu einer Größe von 27 bis 30 kD abbauen lässt. Diese einer weiteren Proteolyse nicht zugängliche Proteinform wird als ein gegen Protease beständiger Kern, kurz PrP²⁷⁻³⁰, bezeichnet. Sie entsteht durch Abbau von ca. 67 Aminosäuren am NH₂-Terminus und besteht selbst aus ca. 141 Aminosäuren.

Einige Verfahren zum Nachweis der pathologischen Prion-Isoformen sind bereits bekannt. So beschreiben beispielsweise Barry und Prusiner J. Infect. Dis. 154, 518-521 (1986) einen Westernblot-Test unter Verwendung eines monoklonalen Anti-Prionenprotein Antikörpers (MAK) 13A5. Dieser Hamster-PrP spezifische MAK wurde in Mäusen gewonnen, die mit gereinigtem, denaturiertem PrP²⁷⁻³⁰ , isoliert aus Scrapie-infizierten Hamstern, immunisiert worden waren.

Andere Antikörper, die wie der MAK 13A5 sowohl gegen PrP^{C} als auch gegen PrP^{Sc}, sofern dieses denaturiert vorliegt, gerichtet sind, sind aus dem US Patent 4806627 bekannt. Des weiteren sind Immunisierungen mit rekombinanten Prionenproteinen, welche in Bakterien exprimiert worden waren, durchgeführt worden, so beschrieben in Zanusso et al., Proc. Natl. Acad. Sci. USA , 95, 8812-8816 (1998). Ebenso gelang die Herstellung monoklonaler Antikörper mittels Peptidimmunisierung, so beschrieben in Harmeyer et al., J. Gen. Virology, 79, 937-945 (1998) und mittels Nukleinsäure-lmmunisierung, wie in Krasemann et al., J. Biotechnology, 73, 119-129 (1999) erläutert.

Eine weitere Anwendung dieser Antikörper neben dem Western Blot wurde in dem US Patent 4806627 erwähnt, nämlich ein so genannter ELISA (enzyme-linked immunosorbent assay). In diesem ELISA wurden Prionen, welche auf einer Mikrotiter-Platte fixiert waren, von dem MAK 3F4 gebunden und dieser dann mittels eines Zweitantikörpers, der über ein an ihm gekoppeltes Enzym eine Farbstoffreaktion katalysiert, detektiert.

In all diesen Nachweisverfahren wird die Probe mit dem Enzym Proteinase K vorbehandelt, um in der Probe befindliches, normales Prion-Protein zu entfernen und somit den alleinigen Nachweis des Protease-resistenten, pathogenen Prion-Proteins zu gewährleisten, da ja die Antikörper auch das normale Prion-Protein mit hoher Affinität binden können.

Schließlich ist auch schon aus der internationalen Patentanmeldung WO 98/37411 ein Nachweisverfahren bekannt, mit dem der Nachweis der pathogenen Konformation des Prion-Proteins in einer Probe möglich ist. Die Probe wird dabei in zwei Portionen aufgeteilt und die erste Portion an einen festen Träger gebunden und dann mit einem markierten Antikörper kontaktiert. Dieser Antikörper bindet an die nicht-pathogene Form des Prionenproteins mit höherer Affinität als an die nicht denaturierte, pathogene Form des Proteins. Die zweite Portion der Probe wird dann einer Behandlung unterzogen, durch die sich die Konformation des pathogenen Prionenproteins ändert und dadurch sich die Zugänglichkeit und damit die Affinität für den markierten Antikörper drastisch erhöht. Die so behandelte zweite Probe wird dann mit einem zweiten Träger in Berührung gebracht und mit einem markierten Antikörper umgesetzt. Dann werden die Mengen des in der ersten und in der zweiten Portion gebundenen markierten Antikörpers gemessen und miteinander verglichen. Der Unterschied zwischen beiden Messergebnissen zeigt an, ob die pathogene Form des Prionenproteins in der Probe vorhanden war. Dieses Nachweis- verfahren wird konformationsabhängiger Immunoassay genannt, im Englischen mit der Abkürzung CDI für conformation-dependent Immunoassay versehen. Die Empfindlichkeit des CDIs lässt sich erhöhen, wenn die Probe einer Vorbehandlung mit einem proteolytischen Enzym, beispielsweise der Proteinase K oder der Dispase unterworfen wird. Durch die Behandlung mit Proteasen werden PrP^{c} und nicht-relevante Proteine in der Probe zerstört und das Protease-resistente PrP²⁷⁻³⁰ bleibt in der Probe übrig.

Die Untersuchung von humanem Blutplasma auf Anwesenheit des pathogenen Prionenproteins erfordert sehr empfindliche und spezifische Nachweissysteme, die sich auch für die Automatisierung eignen. Erschwert wird der Nachweis dadurch, dass die physiologischen Grundlagen für die pathologische Wirkung von Prionen noch nicht bekannt sind.

In der deutschen Patentanmeldung 101 52 677.6 sind kürzlich erstmals Antikörper zum spezifischen Nachweis von pathogenen Prionen humanen Ursprungs beschrieben worden. Dabei werden monoklonale Antikörper aus den Hybridomazelllinien DSM ACC 2522, DSM ACC 2523 und DSM ACC 2524 eingesetzt, die in einem konformationsabhängigen Immunoassayverfahren die nicht-pathologische Konformation von humanen Prionenproteinen von der pathologischen Konformation humaner Prionenproteine unterscheiden können.

Trotz aller bisher entwickelten Nachweisverfahren von pathogenen Prionen besteht weiterhin ein erhebliches Bedürfnis, zusätzliche, schnell durchführbare, zuverlässige und hochempfindliche Nachweisverfahren für pathogene Prionen zur Verfügung zu haben.

Überraschenderweise wurde gefunden, dass bei der Umsetzung eines Gemisches aus Prionenprotein mit pathologischer und nicht-pathologischer Konformation mit einem chemischen Agens, das geeignet ist zusätzliche kovalente Bindungen in dem Prionenprotein zu erzeugen, konformationsabhängig Moleküle entstehen, die massenspektroskopisch unterscheidbare Signale erzeugen.

Ein auf diesem Befund aufbauendes Verfahren zum Nachweis von pathogenen Prionenproteinen in einer Probe einer Körperflüssigkeit humanen oder tierischen Ursprungs, enthaltend ein PrP-Protein, das eine erste natürliche, nicht-pathologische Konformation, PrP^{c}, und eine zweite pathologische Konformation, PrP^{Sc} genannt, einnimmt kann so durchgeführt werden, dass man
- in einer Probe einer Körperflüssigkeit, welche auch chemisch modifiziert sein kann,
- die Prionenproteine mit einem chemischen Agens unter Bildung kovalenter Bindungen umsetzt und
- die dadurch chemisch modifizierten Prionenproteine massenspektroskopisch untersucht, wobei bei Vorliegen von pathogenen Prionen im Massenspektrum mindestens ein weiterer Peak beobachtet wird.

Um die Empfindlichkeit zu steigern und mögliche Störungen auszuschalten, kann man
- die Probe mit einer Prionenproteine adsorbierenden Trägersubstanz kontaktieren,
- die adsorbierten Prionenproteine von der übrigen Probe trennen,
- die Prionenproteine in adsorbiertem Zustand oder nach Freisetzung mit einem chemischen Agens unter Bildung kovalenter Bindungen umsetzen, und
- die dadurch chemisch modifizierten Prionenproteine massenspektroskopisch untersuchen.

Bei Vorliegen von pathogenen Prionen wird dabei im Massenspektrum, gegenüber dem nicht-pathogenen Prionenprotein mindestens ein weiterer Peak beobachtet.

Zur Adsorption kann als Trägersubstanz Agarose, ein Chromatographieharz, eine Mikrotiterplatte oder eine Nitrocellulose- oder Polyamidmembran eingesetzt werden. Diese Trägersubstanz ist mit einem Bindemittel für Prionen beschichtet.

Hierfür kommen Lysozym oder eines seiner Fragmente, ein Prionen bindender monoklonaler oder polyklonaler Antikörper oder eines seiner Fragmente oder eine andere Verbindung in Betracht, die Prionen bindende Liganden aufweist.

Ein derartiger Träger wird mit einer auf das Vorliegen pathogener Prionen zu untersuchenden Körperflüssigkeit, zum Beispiel Blut, Serum, Plasma, Urin, Milch oder fluidisierte Organe wie Hirngewebe, Lymphknoten, Mandeln oder Muskeln in Kontakt gebracht werden. Die auf dem Träger fixierten Prionen können dann für das erfindungsgemäße Nachweisverfahren direkt oder nach Elution der Prionen von dem Träger nachgewiesen werden.

Das erfindungsgemäße Nachweisverfahren beruht nun auf der Erkenntnis, dass natürliche, nicht-pathologische Prionen sich bei gleicher molekularer Zusammensetzung von der pathologischen Konformation der Prionen, PrP^{Sc} durch ihre Raumstruktur unterscheiden und deshalb qualitativ und quantitativ andere funktionelle Gruppen an ihrer Oberfläche für eine Reaktion mit einem chemischen Agens präsentieren. Bringt man deshalb ein Gemisch pathologischer und nicht-pathologischer Prionen z. B. mit einem Oxidations- oder Reduktionsmittel, mit einem Alkylierungs- oder Acylierungsmittel zusammen, dann wird das chemische Agens qualitativ und quantitativ unterschiedliche funktionelle Gruppen an der Prionenoberfläche antreffen und deshalb eine unterschiedliche Anzahl von Bindungen mit dem nicht-pathologischen Prion einerseits und mit dem pathologischen Prion andererseits eingehen. Als Folge davon unterscheiden sich die Massen der mit einem bestimmten chemischen Agens erhaltenen Reaktionsprodukte von nicht-pathologischen Prionen von denen pathologischer Prionen so deutlich, dass sie massenspektroskopisch unterschieden werden können.

Enthält die zu untersuchende Probe einer Körperflüssigkeit humanen oder tierischen Ursprungs nur nicht-pathologische Prionen, dann ist im Massenspektrum nur ein einheitlicher Peak oder eine Gruppe von zusammengehörigen Peaks zu erkennen. Sind in der zu untersuchenden Probe allerdings auch pathologische Prionen enthalten, erhält man ein abweichendes Massenspektrogramm. Es tritt dann neben dem Peak, der für nicht-pathologische Prionen charakteristisch ist, mindestens ein weiterer Peak oder auch eine Gruppe von weiteren Peaks auf, die für die Umsetzung des eingesetzten chemischen Agens mit dem pathologischen Prion charakteristisch ist.

Als chemische Agentien kommen alle Substanzen in Frage, die in der Lage sind, mit den an der Oberfläche von Prionen auftretenden funktionellen Gruppen zu reagieren. Das können sowohl Oxidations- als auch Reduktionsmittel sein. Geeignete Oxidationsmittel sind beispielsweise H₂O₂, Cu⁺⁺/Ascorbat oder Fe⁺⁺⁺/Ascorbat, während als Reduktionsmittel z.B. NaBH₄ eingesetzt werden kann.

Massenunterschiede zwischen den Reaktionsprodukten nicht-pathogener Prionen und pathogener Prionen lassen sich aber auch durch die Umsetzung mit Alkylierungs- und Acylierungsmitteln erzielen. Ein geeignetes Alkylierungs- mittel ist z.B. Formaldehyd, während als Acylierungsmittel Dicarboxylsäure- anhydride, wie Succinsäureanhydrid bevorzugt werden.

Die Prionen zeigen an ihrer Oberfläche aber auch spezielle Seitenketten, die durch Cystein- oder Methionin-, durch Asparaginsäure- oder Glutaminsäure- oder durch Asparagin- oder Glutamin- sowie Lysin- oder Argininreste charakterisiert sind. Als Agentien, die für die Umsetzung mit derartigen Seitenketten in Betracht kommen, sind zum Beispiel Maleinsäureanhydrid (zur Modifikation der SH-Cysteinreste), Diazoacetamid (zur Reaktion mit Glutaminsäure, Asparaginsäureestern und Cysteinresten) und 1,2-Cyclohexandion (zur Umsetzung mit Argininresten) zu nennen.

Die Zuverlässigkeit und Empfindlichkeit des erfindungsgemäßen Nachweisverfahrens konnte dadurch gezeigt werden, dass Gruppen von 10 und 100 Plasmaproben mit ganz geringen Mengen nicht-pathogener und pathogener Prionen versetzt wurden und in allen Fällen ein sicherer Nachweis der pathogenen Prionen neben den nicht-pathogenen Prionen im Massen- spektrogramm möglich war.

Die Durchführung des erfindungsgemäßen Nachweisverfahrens wird durch die nachfolgenden Beispiele veranschaulicht:

### Detektion von Prionenproteinen nach Oxidation

### Beispiel 1:

Plasmaproteinlösungen werden mit Prionenproteinen unterschiedlicher Konformation und unterschiedlichen Ursprungs versetzt. Die Prionenproteine werden in nano- bis femto-molare Konzentration verdünnt. Die Prionenproteine werden durch eine Mixtur von prionspezifischen Antikörpern immunpräzipitiert. Die immunpräzipitierten Proteine werden gelöst (10 mg/ml Protein in Oxidationspuffer ( 50 mM Hepes Puffer, pH 7,4; 100mM KCI; 10 mM MgCl₂)) und anschließend mittels Metall Katalysierter Oxidation (MKO) behandelt. Dazu wird 750 µl Proteinlösung mit 25 mM Ascorbinsäure und 100µM FeCl₃ versetzt. Der Reaktionsansatz wird bei 37°C für 12 h inkubiert und anschließend die Oxidationsreaktion mittels Zugabe von EDTA Lösung gestoppt. Anschließend werden die Prionenproteine massenspektrometrisch charakterisiert. Dabei wird ein Priontyp spezifisches Chromatogramm erhalten.

### Beispiel 2:

Plasmaproteinlösungen (10 mg/ml Protein in Oxidationspuffer ( 50 mM Hepes Puffer, pH 7,4; 100mM KCI; 10 mM MgCl₂)) werden mit Prionenproteinen unterschiedlicher Konformation und unterschiedlichen Ursprungs versetzt. Die Prionenproteine werden in nano- bis femto-molare Konzentration verdünnt. Die Proteinlösung wird anschließend mittels Metall Katalysierter Oxidation (MKO) behandelt. Dazu wird 750 µl Proteinlösung mit 25 mM Ascorbinsäure und 100µM FeCl₃ versetzt. Der Reaktionsansatz wird bei 37°C für 12 h inkubiert und anschließend die Oxidationsreaktion mittels Zugabe von EDTA Lösung gestoppt. Die Prionenproteine werden durch eine Mixtur von prionspezifischen Antikörpern immunpräzipitiert und anschließend massenspektrometrisch charakterisiert. Dabei wird ein Priontyp spezifisches Chromatogramm erhalten.

### Beispiel 3:

Plasmaproteinlösungen werden mit Prionenproteinen unterschiedlicher Konformation und unterschiedlichen Ursprungs versetzt. Die Prionenproteine werden in nano- bis femto-molare Konzentration verdünnt. Die Prionenproteine werden durch eine Mixtur von prionspezifischen Antikörpern an einen Träger gebunden. Die gebundenen Proteine werden auf dem Träger mittels Oxidationspuffer ( 50 mM Hepes Puffer, pH 7,4; 100mM KCI; 10 mM MgCl₂) und Metall Katalysierter Oxidation (MKO) behandelt. Dazu wird der Träger mit 25 mM Ascorbinsäure und 100µM FeCl₃ versetzt. Der Reaktionsansatz wird bei 37°C für 12 h inkubiert und anschließend die Oxidationsreaktion mittels Zugabe von EDTA Lösung gestoppt. Anschließend werden die gebundenen und oxidierten Prionen massenspektrometrisch charakterisiert. Dabei wird ein Priontyp spezifisches Chromatogramm erhalten.

### Beispiel 4:

Plasmaproteinlösungen werden mit Prionenproteinen unterschiedlicher Konformation und unterschiedlichen Ursprungs versetzt. Die Prionenproteine werden in nano- bis femto-molare Konzentration verdünnt. Die Prionenproteine werden durch eine Mixtur von prionspezifischen Antikörpern immunpräzipitiert. Die immunpräzipitierten Proteine werden gelöst (10 mg/ml Protein in Oxidationspuffer ( 50 mM Hepes Puffer, pH 7,4; 100mM KCI; 10 mM MgCl₂)) und anschließend mittels Metall Katalysierter Oxidation (MKO) behandelt. Dazu wird 750 µl Proteinlösung mit 25 mM Ascorbinsäure und 100µM FeCl₃ versetzt. Der Reaktionsansatz wird bei 37°C für 12 h inkubiert und anschließend die Oxidationsreaktion mittels Zugabe von EDTA Lösung gestoppt. Die oxidierten Proteine werden mit 2,4-Dinitrophenylhydrazin derivatisiert. Anschließend werden die Prionenproteine massenspektrometrisch charakterisiert. Dabei wird ein Priontyp spezifisches Chromatogramm erhalten.

## Patentansprüche

1. Verfahren zum Nachweis von pathogenen Prionenproteinen in einer Probe einer Körperflüssigkeit humanen oder tierischen Ursprungs, enthaltend ein PrP-Protein, das eine erste natürliche, nicht-pathologische Konformation, PrP^{c}, und eine zweite pathologische Konformation, PrP^{Sc} genannt, einnimmt, **dadurch gekennzeichnet, dass** man
- in einer Probe einer Körperflüssigkeit, welche auch chemisch modifiziert sein kann,
- die Prionenproteine mit einem chemischen Agens unter Bildung kovalenter Bindungen umsetzt und
- die dadurch chemisch modifizierten Prionenproteine massenspektroskopisch untersucht, wobei bei Vorliegen von pathogenen Prionen im Massenspektrum mindestens ein weiterer Peak beobachtet wird.

2. Verfahren nach Anspruch 1, **dadurch gekennzeichnet, dass** man
- die Probe mit einer Prionenproteine adsorbierenden Trägersubstanz kontaktiert,
- die adsorbierten Prionenproteine von der übrigen Probe trennt,
- die Prionenproteine in adsorbiertem Zustand oder nach Freisetzung mit einem chemischen Agens unter Bildung kovalenter Bindungen umsetzt.

3. Verfahren nach den Ansprüchen 1 und 2, **dadurch gekennzeichnet, dass** als Trägersubstanz Agarose, ein Chromatographieharz, eine Mikrotiterplatte, oder eine Nitrocellulose- oder Polyamidmembran eingesetzt wird.

4. Verfahren nach den Ansprüchen 1 bis 3, **dadurch gekennzeichnet, dass** die Trägersubstanz mit Lysozym oder einem seiner Fragmente, mit einem polyklonalen oder monoklonalen Antikörper oder einem seiner Fragmente oder mit einer anderen Verbindung beschichtet ist, die Prionenproteinen bindende Liganden aufweist.

5. Verfahren nach den Ansprüchen 1 bis 4, **dadurch gekennzeichnet, dass** als Körperflüssigkeit Blut, Serum, Plasma, Urin, Milch oder fluidisierte Organe wie Hirngewebe, Lymphknoten, Mandeln oder Muskeln verwendet werden.

6. Verfahren nach den Ansprüchen 1 bis 5, **dadurch gekennzeichnet, dass** man die auf dem Träger adsorbierten Prionenproteine für das Nachweisverfahren einsetzt oder die Prionenproteine zunächst von dem Träger eluiert und das Nachweisverfahren mit den eluierten Prionen durchführt.

7. Verfahren nach den Ansprüchen 1 bis 6, **dadurch gekennzeichnet, dass** man die Prionenproteine mit einem chemischen Agens aus der Gruppe der Oxidations- oder Reduktionsmittel umsetzt.

8. Verfahren nach den Ansprüchen 1 bis 6, **dadurch gekennzeichnet, dass** man die Prionenproteine mit einem chemischen Agens aus der Gruppe der Alkylierungsmittel umsetzt.

9. Verfahren nach den Ansprüchen 1 bis 6, **dadurch gekennzeichnet, dass** man die Prionenproteine mit einem chemischen Agens aus der Gruppe der Acylierungsmittel umsetzt.

10. Verfahren nach den Ansprüchen 1 bis 6, **dadurch gekennzeichnet, dass** man die Prionenproteine spezifisch an Cystein und Methionin Seitenketten modifiziert.

11. Verfahren nach den Ansprüchen 1 bis 6, **dadurch gekennzeichnet, dass** man die Prionenproteine spezifisch an Asparaginsäure und Glutaminsäure Seitenketten modifiziert.

12. Verfahren nach den Ansprüchen 1 bis 6, **dadurch gekennzeichnet, dass** man die Prionenproteine spezifisch an Asparagin und Glutamin Seitenketten modifiziert.

13. Verfahren nach den Ansprüchen 1 bis 6, **dadurch gekennzeichnet, dass** man die Prionenproteine spezifisch an Lysin und Arginin Seitenketten modifiziert.
